# EUROPEAN PATENT APPLICATION

(11) **EP 4 115 924 A1**
(43) Date of publication of application: **11.01.2023**
(21) Application number: 21755687.7
(22) Date of filing: 19.02.2021
(51) Int. Cl.: A61M 5/145, A61M 5/142

(54) **ELECTROCHEMICAL PUMP AND DELIVERY DEVICE**

(30) Priority: 21.02.2020 US 202062979772 P
(71) Applicant: Micromed Co., Ltd., Taipei City, Taiwan 115 (TW)
(72) Inventor: LI, Po-Ying, New Taipei City, Taiwan 241 (CN); CHENG, Tsung-Chieh, New Taipei City, Taiwan 241 (CN); LAI, Jiunn-Ru, Kaohsiung City, Taiwan 807618 (CN)
(74) Representative: Reichert & Lindner Partnerschaft Patentanwälte
(86) International application number: PCT/CN2021/076805
(87) International publication number: WO 2021/164721

(57) **Abstract**

An electrochemical pump adopts a hybrid pulse to intermittently activate an electrochemical reaction. The abovementioned electrochemical pump can save power effectively to prolong the lifetime of the electrochemical pump. In addition, an electrochemical pump enhances the bonding strength between electrodes and a substrate by covering edges of the electrodes with an insulating layer to avoid electrode delamination caused by high-power electrochemical reactions. A delivery device using the abovementioned electrochemical pump is also disclosed.

## Description

### BACKGROUND OF THE INVENTION

### 1. FIELD OF THE INVENTION

The present invention relates to a pump and a delivery device, particularly to an electrochemical pump generating driving force electrochemically and a delivery device using the same.

### 2. DESCRIPTION OF THE PRIOR ART

Injection, such as hypodermic injection or intravenous injection, is a common-seen method of delivering medicine into bodies. At present, pen-type injectors and electronically controlled injectors, such as patch-type injectors, wearable injectors, and implanted injectors, have been developed to enable users to inject medicine by themselves. While the conventional pen-type injectors utilize springs to generate driving force to deliver medicine, the pen-type injector can only inject a small amount of medicine instead of a large amount thereof.

One conventional electronically controlled injector delivers medicine via the driving force provided by a motor. The injection time and injection dosage can be adjusted via controlling motor rotation. However, the electronically controlled injector with the motor is difficult to be miniaturized and is inconvenient for the patient for long-term carrying/wearing. Another conventional electronically controlled injector is only suitable for delivering insulin. For other macromolecular drugs (or biologics), such as monoclonal antibody, hormone, and growth factor, the conventional electronically controlled injectors are challenging of providing sufficient driving forces, especially in the case of delivering drugs in pre-filled containers (e.g., pre-filled syringe or pre-filled cartridge) due to the airtight seal between the rubber plunger and the glass of these containers. Further, to extend the lifetime of the electronically controlled injector devices, power administration is also critical and requires cutting-edge solutions.

Accordingly, it is highly desirable to provide a new pump technology capable of overcoming the abovementioned problems.

### SUMMARY OF THE INVENTION

The present invention provides an electrochemical pump and a delivery device thereof, wherein a hybrid pulse is used to control the electrochemical reaction, whereby power is effectively saved, and the lifetime of the electrochemical pump is significantly prolonged.

The present invention provides another electrochemical pump and a delivery device thereof, wherein edges of electrodes are covered with an insulating layer to protect the bonding between the electrodes and a substrate to allow high-power electrochemical reaction for high flow rate and/or large driving force.

In one embodiment, the electrochemical pump of the present invention comprises a substrate, a plurality of electrodes, a dam, and a control circuit. The substrate has an electrode region. The electrodes are disposed in the electrode region. The dam encircles the electrode region and defines a containing space. The containing space stores an electrochemical liquid. The control circuit is electrically connected with the electrodes and uses a pulse signal to selectively activate an electrochemical reaction on surfaces of the electrodes, wherein an enabling pulse of the pulse signal includes a plurality of sub-enabling pulses.

In one embodiment, the delivery device of the present invention comprises an electrochemical pump, a container, and a delivery connector. The electrochemical pump comprises a substrate, a plurality of electrodes, a dam, and a control circuit. The substrate has an electrode region. The electrodes are disposed in the electrode region. The dam encircles the electrode region and defines a containing space. The containing space stores an electrochemical liquid. The control circuit is electrically connected with the electrodes and uses a pulse signal to selectively activate an electrochemical reaction on surfaces of the electrodes, wherein an enabling pulse of the pulse signal includes a plurality of sub-enabling pulses. The container includes a sealing element and a piston. A liquid, which is to be delivered, is stored between the sealing element and the piston. The container is connected with the electrochemical pump, whereby an airtight room is defined between the piston and the containing space of the electrochemical pump. The delivery connector includes a tube, a puncture element, and a delivery element. The puncture element is connected with one end of the tube and used to puncture the sealing element of the container, whereby the container communicates with exterior through the puncture element. The delivery element is connected with another end of the tube and disposed on an object. The delivered liquid is pushed by the piston to arrive at the object through the puncture element, the tube, and the delivery element.

In another embodiment, the electrochemical pump of the present invention comprises a substrate, a plurality of electrodes, an insulating layer, a dam, and a control circuit. The substrate has an electrode region. The electrodes are disposed in the electrode region. The insulating layer covers edges of the electrodes, and a portion of the electrodes is exposed. The dam encircles the electrode region and defines a containing space. The containing space stores an electrochemical liquid. The control circuit is electrically connected with the electrodes, selectively activating an electrochemical reaction on surfaces of the electrodes.

### BRIEF DESCRIPTION OF THE DRAWINGS

The foregoing conceptions and their accompanying advantages of this invention will become more readily appreciated after being better understood by referring to the following detailed description, in conjunction with the accompanying drawings, wherein:
Fig. 1 is a diagram schematically showing a delivery device according to a first embodiment of the present invention;
Fig. 2 is a diagram schematically showing an electrochemical pump of a delivery device according to a second embodiment of the present invention;
Fig. 3 is a diagram schematically showing an electrochemical pump of a delivery device according to a third embodiment of the present invention;
Fig. 4 is a diagram schematically showing a substrate and electrodes of a delivery device according to a fourth embodiment of the present invention;
Fig. 5 is a diagram schematically showing a hybrid pulse signal of a delivery device according to one embodiment of the present invention; and
Fig. 6 is a diagram schematically showing a delivery device according to a fifth embodiment of the present invention.

### DESCRIPTION OF THE PREFERRED EMBODIMENTS

The present invention will be described in detail with embodiments and attached drawings below. However, these embodiments are only to exemplify the present invention but not to limit the scope of the present invention. In addition to the embodiments described in the specification, the present invention also applies to other embodiments. Further, any modification, variation, or substitution, which can be easily made by the persons skilled in that art according to the embodiment of the present invention, is to be also included within the scope of the present invention, which is based on the claims stated below. Although many special details are provided herein to make the readers more fully understand the present invention, the present invention can still be practiced under a condition that these special details are partially or completely omitted. Besides, the elements or steps, which are well known by the persons skilled in the art, are not described herein lest the present invention be limited unnecessarily. Similar or identical elements are denoted with similar or identical symbols in the drawings. It should be noted that the drawings are only to depict the present invention schematically but not to show the real dimensions or quantities of the present invention. Besides, matterless details are not necessarily depicted in the drawings to achieve conciseness.

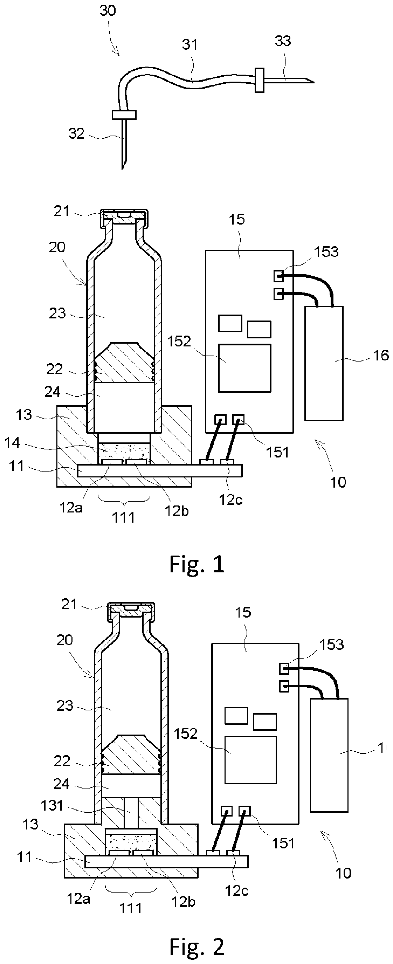

Refer to Fig. 1. In one embodiment, the delivery device of the present invention comprises an electrochemical pump 10, a container 20, and a delivery connector 30. The container 20 includes a sealing element 21 and a piston 22. A storage room 23 is formed between the sealing element 21 and the piston 22 for storing a liquid, which is to be delivered. For example, the liquid to be delivered may be a drug or biologics such as monoclonal antibody, targeted drugs, antibody drugs and other biosimilar drugs. However, the liquid is not limited to be water-based fluid. Liquids may also be small molecule drugs. The liquid may be a solvent-based fluid (such as DMSO) or an oilbased fluid (such as corn oil). In one embodiment, the container 20 may be a pre-filled container (pre-filled syringe or pre-filled cartridge).

The delivery connector 30 includes a tube 31, a puncture element 32, and a delivery element 33. The puncture element 32 is connected to one end of the tube 31 and used to puncture the sealing element 21 of the container 20, whereby the storage room 23 of the container 20 communicate with the exterior of the container 20 through puncture element 32. The delivery element 33 is connected to another end of the tube 31 and is to be disposed on an object. For example, the delivery element 33 may be inserted or implanted hypodermically, subcutaneously, intramuscularly, intravenously, or intraperitoneally. However, the delivery element 33 is not limited to be disposed in the abovementioned regions but may also be disposed on another appropriate region. The delivery element 33 shown in Fig. 1 is a needle-like structure. However, the delivery element 33 is not limited to a needle-like structure but may be a connector, which is to be connected with another syringe or delivery instrument. Thereby, the delivery device of the present invention may be remote from the position where the drug is to be delivered. For example, the delivery device may be worn on the arm, abdomen, thigh, or hips of a patient, and the needle is inserted into the adjacent area of the aforementioned position of the patient.

With the abovementioned structure, after the puncture element 32 of the delivery connector 30 punctures the sealing element 21 of the container 20, the liquid (such as a drug) stored inside the storage room 23 for delivery may be delivered to the object by pushing the piston 22 through the puncture element 32, the tube 31, and the delivery element 33.

The structure of the electrochemical pump 10 will be described in detail below. The electrochemical pump 10 of the present invention comprises a substrate 11, an electrode 12a, an electrode 12b, a dam 13, and a control circuit 15. The substrate 11 has an electrode region 111, and the electrodes 12a and 12b are disposed in the electrode region 111 of the substrate 11. In one embodiment, the substrate 11 is made of glass, quartz, ceramic, semiconductor material, or plastic. For example, the ceramic may be aluminum oxide, titanium oxide, etc.; the semiconductor material may be silicon. The dam 13 encircles the electrode region 111 of the substrate 11 and defines a containing space for storing an electrochemical liquid 14. The control circuit 15 is electrically connected with the electrodes 12a and 12b. For example, the substrate 11 includes a plurality of electric-conduction contacts 12c, and the control circuit 15 includes electric-conduction contacts 151. Via leads or another appropriate means (such as connector or pogo pin), the electric-conduction contacts 151 of the control circuit 15 are electrically connected with the plurality of electric-conduction contacts 12c. Thereby, the control circuit 15 is electrically connected with the electrodes 12a and 12b. The control circuit 15 includes necessary electronic elements 152 (such as a microcontroller and passive elements) and electric-conduction contacts 153 for electric conduction with a power supply 16 (such as a battery). Neither the detailed structure of the control circuit 15 nor the connection means of the power supply 16 is the primary technical characteristic of the present invention. Therefore, they will not be repeated herein.

The container 20 is connected with the electrochemical pump 10, and an airtight room 24 is defined between the piston 22 of the container 20 and the containing space formed by the dam 13. For example, an engagement structure corresponding to the container 20 is formed in the dam 13; while the container 20 is disposed into the engagement structure of the dam 13, the container 20 and the dam 13 define an airtight room 24 between the piston 22 and the electrochemical liquid 14. The control circuit 15 selectively supplies power to the electrodes 12a and 12b to selectively enable an electrochemical reaction on the surfaces of the electrodes 12a and 12b, thereby generating gas. This additional gas increases the pressure inside the airtight room 24 and thus pushes the piston 22 to move.

In the embodiment shown in Fig. 1, the dam 13 contacts the outer wall of the container 20 to form the airtight room 24. However, the present invention is not limited by this embodiment. Refer to Fig. 2. In one embodiment, the dam 13 contacts the inner wall of the container 20 to form the airtight room 24. In the embodiment shown in Fig. 2, the airtight room 24 communicates with the containing space formed by the dam 13 through a passage 131. Thereby, the gas generated by the electrochemical reaction enters the airtight room 24 through the passage 131 to increase the pressure inside the airtight room 24.

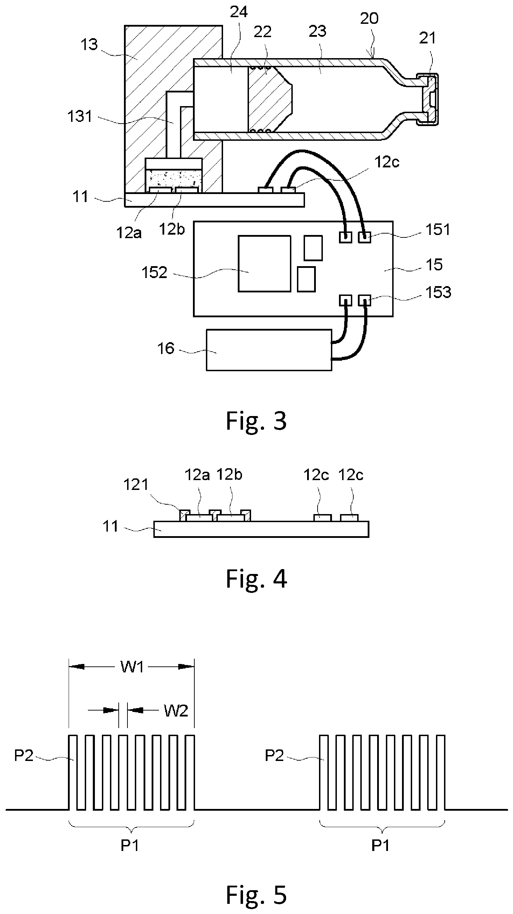

It is easily understood: the design that the passage 131 is used to communicate the airtight room 24 and the containing space formed by the dam 13 facilitates different designs of the relative position of the container 20 and the substrate 11. In the embodiments shown in Fig. 1 and Fig. 2, the container 20 is vertical to the substrate 11. However, the present invention is not limited by the two embodiments. Refer to Fig. 3. In one embodiment, the container 20 is parallel to the substrate 11. It should be noted that the present invention is not limited by the embodiments in which the container 20 and the electrochemical pump 10 are directly connected to each other. In other embodiments, appropriate adapters may be used to connect the container 20 and the dam 13, whereby different containers or layouts may be used.

Also, refer to Fig. 4. In one embodiment, the electrochemical pump 10 further comprises an insulating layer 121. The insulating layer 121 covers the edges of the electrodes 12a and 12b and reveals a portion of the electrodes 12a and 12b. With the abovementioned structure, the insulating layer 121 enhances the bonding strength between the substrate 11 and the electrodes 12a and 12b, decreases the chance that gas enters the interfaces between the substrate 11 and the electrodes 12a and 12b, and thus prevents electrode delamination in a high-power electrochemical reaction. In one embodiment, the insulating layer 121 is made of epoxy (such as solder mask, SU-8), photo-patternable polymer, photo-patternable silicone, glass, ceramic, or plastic. For example, the photo-patternable polymer may be photoresist, photo-patternable polyimide, or photo-patternable adhesives. In one embodiment, the insulating layer 121 can be formed by screen printing, semiconductor manufacturing, or sintering.

As mentioned above, the control circuit 15 selectively supplies power to enable an electrochemical reaction and generate gas on the surfaces of the electrodes 12a and 12b. Refer to Fig. 5. In one embodiment, the control circuit 15 uses the pulse signal shown in Fig. 5 to selectively enable an electrochemical reaction on the surfaces of the electrodes 12a and 12b. The pulse signal shown in Fig. 5 includes two enabling pulses P1. It is easily understood that the width W1 of the enabling pulse P1 may be modified according to a target output of the electrochemical pump 10. For example, while the width W1 of the enabling pulse P1 is larger, the triggered electrochemical reaction is longer, and more gas is generated. Contrarily, while the width W1 of the enabling pulse P1 is smaller, the triggered electrochemical reaction is shorter, and less gas is generated. It is easily understood: because the response of the electrochemical reaction is slower in comparison with the change of electric signal, gas is still generated between two enabling pulses P1. Therefore, appropriately adjusting the width W1 of the enabling pulse P1 may generate a required amount of gas and save energy. In one embodiment, the pulse signal in the present invention can be realized by pulse width modulation (PWM) technology. It is easily understood that setting the widths W1 of the enabling pulses P1 to be the same can also generate a predetermined amount of gas.

Particularly, in one embodiment, the enabling pulse P1 includes a plurality of sub-enabling pulses P2. In other words, while the enabling pulse P1 enables the electrochemical reaction, it does not activate the electrochemical reaction continuously but intermittently. Similarly, as mentioned above, gas is still generated between two sub-enabling pulses P2. Therefore, the enabling pulse P1 formed by a plurality of sub-enabling pulses P2 may save energy furthermore. In one embodiment, the width W2 of the sub-enabling pulses P2 may be the same. It is easily understood that the width W2 of the sub-enabling pulses P2 may be modified to adjust the target output of the electrochemical pump 10.

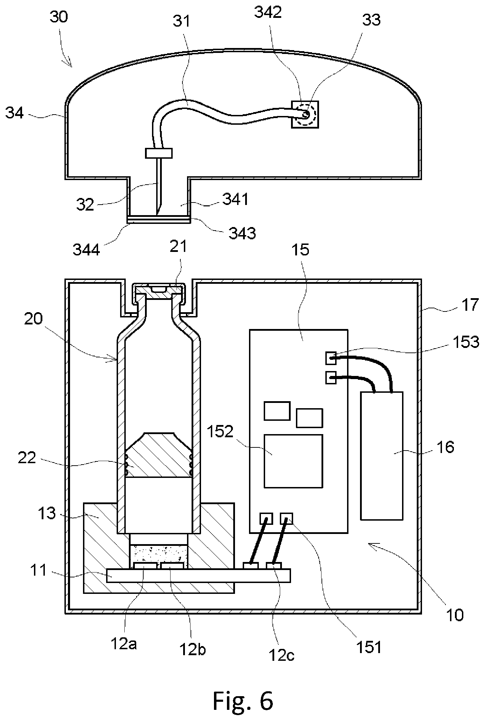

As mentioned above, one of the applications of the delivery device of the present invention is to deliver medicine to an object. Therefore, how to guarantee sterilization of the delivery path between the container 20 and the object is an important subject. Refer to Fig. 6 for the solution to the abovementioned problem. In one embodiment, the delivery connector 30 further comprises a casing 34, and the tube 31, the puncture element 32, and the delivery element 33 are arranged in the casing 34. The casing 34 includes a first opening 341 and a second opening 342, wherein the puncture element 32 is corresponding to the first opening 341, and the delivery element 33 is corresponding to the second opening 342. The first opening 341 and the second opening 342 are respectively sealed by sealing membranes 343. Then, the delivery connector 30 having the abovementioned structure is sterilized. Thus, the interior of the casing 34 is maintained in a sterilized state. In other words, the tube 31, the puncture element 32, and the delivery element 33 are all in a sterilized state. In this embodiment, the electrochemical pump 10 and the container 20 are disposed inside a housing 17. While the present invention is to be used, the delivery connector 30 and a housing 17 are correspondingly assembled together. Thus, the puncture element 32 punctures the sealing membrane 343 on the first opening 341 and the sealing element 21 of the container 20. Similarly, the delivery element 33 punctures the sealing membrane on the second opening 342 and then is implanted into an appropriate position of the object. Thereby, the drug delivery path between the container 20 and the object is maintained in a sterilized state. In one embodiment, the sealing membrane on the first opening 341 and on the second opening 342 can be removed before the puncture element 32 is to puncture the sealing element 21, and the delivery element 33 is implanted into the object.

According to the abovementioned structure, it should be explained that the delivery connector 30, the container 20, and the electrochemical pump 10 may be fabricated by different manufacturers respectively and then assembled together to form a complete product. Thus, the high temperature used to sterilize the delivery connector 30 would not affect the stability of the drug in the container 20. Further, the demand for the cleanness of the environment where the parts are assembled together is lowered.

It is easily understood that the outer surface of the sealing membrane 343 will be polluted after sterilization because it may contact the external environment. Therefore, a sterilizing process, such as swabbing the outer surface of the sealing membrane 343, may be used to decrease the risk of the pollution of the drug delivery path. Refer to Fig. 6 for an embodiment that can simplify the sterilization operation. In Fig. 6, the delivery connector 30 further comprises a protection layer 344, which is disposed on the outer surface of the sealing membrane 343. With the abovementioned structure, while the present invention is to be used, only removing the protection layer 344 is sufficient to guarantee the sterilized state of the sealing membrane 343. Therefore, the protection layer 344 can secure the sterilization of the sealing membrane 343 and simplify the operation process of using the present invention.

In conclusion, the electrochemical pump and the delivery device of the present invention use hybrid pulses to control an electrochemical reaction, whereby electric energy is effectively saved and the usage time of the electrochemical pump is significantly prolonged. Further, an electrochemical pump and a delivery device of the present invention includes an insulating layer covering the edges of the electrodes to enhance the bonding strength between the electrodes and the substrate and decrease the chance that gas enters the interfaces between the electrodes and the substrate, whereby to prevent electrode delamination.

The embodiments have been described above to demonstrate the technical thoughts and characteristics of the present invention to make the persons skilled in the art understand, make, and use the present invention. However, these embodiments are not intended to limit the scope of the present invention. Any equivalent modification or variation according to the spirit of the present invention is to be also included in the scope of the present invention.

## Claims

1. An electrochemical pump comprising:
a substrate having an electrode region;
a plurality of electrodes disposed in the electrode region;
a dam encircling the electrode region to define a containing space, wherein the containing space stores an electrochemical liquid; and
a control circuit electrically connected with the electrodes and using a pulse signal to selectively enable an electrochemical reaction on surfaces of the electrodes, wherein an enabling pulse of the pulse signal includes a plurality of sub-enabling pulses.

2. The electrochemical pump according to claim 1, wherein a width of the enabling pulse is adjusted according to a target output of the electrochemical pump.

3. The electrochemical pump according to claim 1, wherein the pulse signal includes a plurality of the enabling pulses, and widths of the enabling pulses are the same.

4. The electrochemical pump according to claim 1, wherein widths of the sub-enabling pulses are adjusted according to a target output of the electrochemical pump.

5. The electrochemical pump according to claim 1, wherein widths of the sub-enabling pulses are the same.

6. The electrochemical pump according to claim 1, wherein the substrate is made of glass, quartz, ceramic, semiconductor material, or plastic.

7. The electrochemical pump according to claim 1, wherein the substrate is made of glass, quartz, aluminum oxide, titanium oxide, silicon, or plastic.

8. The electrochemical pump according to claim 1 further comprising:
an insulating layer covering edges of the electrodes, wherein a portion of the electrodes is exposed.

9. The electrochemical pump according to claim 8, wherein the insulating layer is made of epoxy, photo-patternable polymer, photo-patternable silicone, glass, ceramic, or plastic.

10. The electrochemical pump according to claim 8, wherein the insulating layer is made of photoresist, photo-patternable polyimide, and photo-patternable adhesives.

11. A delivery device comprising:
an electrochemical pump comprising:
a substrate having an electrode region;
a plurality of electrodes disposed in the electrode region;
a dam encircling the electrode region to define a containing space, wherein the containing space stores an electrochemical liquid; and
a control circuit electrically connected with the electrodes and using a pulse signal to selectively enable an electrochemical reaction on surfaces of the electrodes, wherein an enabling pulse of the pulse signal includes a plurality of sub-enabling pulses;
a container including a sealing element and a piston, wherein a liquid, which is to be delivered, is stored between the sealing element and the piston, and wherein the container is connected with the electrochemical pump to define an airtight room between the piston and the containing space of the electrochemical pump; and
a delivery connector including:
a tube;
a puncture element connected with one end of the tube and used to puncture the sealing element of the container to make the container communicate with exterior through the puncture element; and
a delivery element connected with another end of the tube and to be disposed on an object, wherein the piston pushes the liquid to deliver the liquid to the object through the puncture element, the tube, and the delivery element.

12. The delivery device according to claim 11, wherein the delivery connector further comprises a casing with a first opening and a second opening, wherein the tube, the puncture element, and the delivery element are disposed in the casing, wherein the puncture element and the delivery element are respectively corresponding to the first opening and the second opening, and wherein sealing membranes respectively seal the first opening and the second opening to maintain the interior of the casing at a sterilized state.

13. The delivery device according to claim 12, wherein the delivery connector further comprises a protection layer disposed on an external surface of the sealing membranes.

14. The delivery device according to claim 11, wherein a width of the enabling pulse is adjusted according to a target output of the electrochemical pump.

15. The delivery device according to claim 11, wherein the pulse signal includes a plurality of the enabling pulses, and widths of the enabling pulses are the same.

16. The delivery device according to claim 11, wherein widths of the sub-enabling pulses are adjusted according to a target output of the electrochemical pump.

17. The delivery device according to claim 11, wherein widths of the sub-enabling pulses are the same.

18. The delivery device according to claim 11, wherein the substrate is made of glass, quartz, ceramic, semiconductor material, or plastic.

19. The delivery device according to claim 11, wherein the substrate is made of glass, quartz, aluminum oxide, titanium oxide, silicon, or plastic.

20. The delivery device according to claim 11, wherein the electrochemical pump further comprising:
an insulating layer covering edges of the electrodes, wherein a portion of the electrodes is exposed.

21. The delivery device according to claim 20, wherein the insulating layer is made of epoxy, photo-patternable polymer, photo-patternable silicone, glass, ceramic, or plastic.

22. The delivery device according to claim 20, wherein the insulating layer is made of photoresist, photo-patternable polyimide, and photo-patternable adhesives.

23. An electrochemical pump comprising:
a substrate having an electrode region;
a plurality of electrodes disposed in the electrode region;
an insulating layer covering edges of the electrodes, wherein a portion of the electrodes is exposed;
a dam encircling the electrode region to define a containing space, wherein the containing space stores an electrochemical liquid; and
a control circuit electrically connected with the electrodes and selectively enabling an electrochemical reaction on surfaces of the electrodes.

24. The electrochemical pump according to claim 23, wherein the substrate is made of glass, quartz, ceramic, semiconductor material, or plastic.

25. The electrochemical pump according to claim 23, wherein the substrate is made of glass, quartz, aluminum oxide, titanium oxide, silicon, or plastic.

26. The electrochemical pump according to claim 23, wherein the insulating layer is made of epoxy, photo-patternable polymer, photo-patternable silicone, glass, ceramic, or plastic.

27. The electrochemical pump according to claim 23, wherein the insulating layer is made of photoresist, photo-patternable polyimide, and photo-patternable adhesives.

28. The electrochemical pump according to claim 23, wherein the control circuit selectively enables an electrochemical reaction on surfaces of the electrodes by a pulse signal, wherein an enabling pulse of the pulse signal includes a plurality of sub-enabling pulses.
